# EUROPEAN PATENT SPECIFICATION

(11) **EP 0 390 945 B1**
(45) Date of publication and mention of the grant of the patent: **18.11.1993**
(21) Application number: 89105955.2
(22) Date of filing: 05.04.1989
(51) Int. Cl.: C07C 37/18, C07C 39/15

(54) **Process for preparing 4,4'''-dihydroxyquater-phenyl or derivatives thereof**
Verfahren zur Herstellung von 4,4'''-Dihydroxyquaterphenyl oder deren Derivaten
Procédé de préparation de 4,4'''-dihydroxyquater-phényle ou leurs dérivés

(43) Date of publication of application: 10.10.1990
(73) Proprietor: Sanko Kaihatsu Kagaku Kenkyusho, Ibaraki-shi Osaka (JP)
(72) Inventor: Saito, Toranosuke, Ibaraki-shi Osaka (JP); Ikemoto, Kenichi, Arao-shi Kumamoto (JP); Sakaguchi, Katsuya, Omuta-shi Fukuoka (JP); Hirakawa, Norio, Tamana-gun Kumamoto (JP)
(74) Representative: Gervasi, Gemma, Dr.

(56) References cited:
- EP-A- 0 220 835

## Description

The present invention relates to a method for preparing 4,4‴-dihydroxyquater-phenyl or derivatives thereof represented by the following general formula (I): 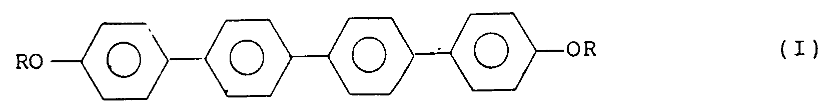
wherein R represents a hydrogen atom, an alkyl group or an acyl group.

### Description of the Prior Art

The compounds represented by the general formula (I) have been expected to be useful as a variety of industrial materials, in particular, materials for liquid crystal compounds of liquid crystal display elements, heat resistant polymeric compounds or modifiers for synthetic rubbers.

As methods for preparing these compounds, there have been proposed the following methods:
(1) A method comprising heating 4′-iodo-4-methoxybiphenyl at 280°C in the presence of a large quantities of copper alloy powders in the nitrogen atmosphere to subject to deiodination-dimerization;
(2) A method comprising reacting 4′-bromo-4-methoxy-biphenyl with magnesium powders and ethyl bromide in ether as a solvent, then adding copper chloride and heating to dimerize the compound. These methods (1) and (2) are, for instance, disclosed in J. Chem. Soc., 1940, pp. 1379-1382.

However, in both methods, a large amount of copper alloy powders or magnesium powders is required, the reaction conditions are too severe, the methods suffer from a variety of problems concerning safety, hygiene or environmental pollution and further the yield of end product is not sufficient.

On the other hand, as for preparation of p-quater-phenyl having no substituents on 4- and 4‴-positions, there are known a method of dimerizing 4-bromobiphenyl with use of sodium formate, palladium on charcoal and a surfactant in a 32% aqueous solution of sodium hydroxide (Synthesis, July, 1978, p.538, Table) and a method of dimerizing 4-bromobiphenyl with use of palladium on charcoal and hexadecyl trimethyl ammonium bromide in a 10% aqueous solution of sodium hydroxide under a pressurised condition of carbon monoxide (EP 0206543, column 14, Example 15). These methods are directed to a process for the preparation of p-quater-phenyl having no nucleus-substituents which is different from the 4,4‴-dihydroxyquater-phenyl and its derivatives of the present invention and also, the yield of end product is very low, the former being 48% and the latter being 10%.

Further it is known a process for carrying out the dehalogeno-dimerization of an aromatic halide compound in the presence of platinum group metal catalyst, carbon monoxide and an alkali metal compound and/or alkaline earth metal compound as disclosed for example in EP-A- 220 835. However since this process involves the reaction of carbon monoxide under a pressurized condition, problems of security and hygiene arise.

### SUMMARY OF THE INVENTION

Accordingly, it is an object of the present invention to provide a method for preparing 4,4‴-dihydroxyquater-phenyl or derivatives thereof, which does not require a large amount of metallic powders and severe reaction conditions, which has no possibility of causing problems concerning safety, hygiene or environmental pollution and which provides end products having high quality with a high yield.

According to the present invention, there is provided a method for preparing 4,4‴-dihydroxyquater-phenyl or derivatives thereof represented by the following general formula (I): 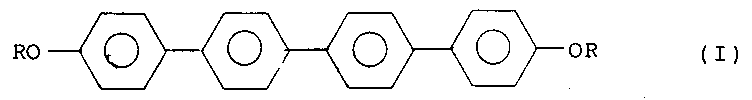
wherein R represents a hydrogen atom or an alkyl having 1 to 4 carbon atoms or an acyl group. The method comprises dehalogenating and dimerizing a 4-hydroxy-4′-halogeno-biphenyl or a derivative thereof represented by the following general formula (II): 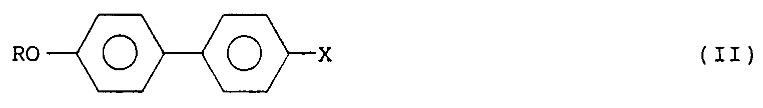
wherein R is the same as that defined above and X represents a halogen atom in an inert organic polar solvent, in the presence of at least one member selected from the group consisting of hydroxides, carbonates or bicarbonates of alkali metals or alkaline earth metals in the absence of carbon monoxide and using palladium as a catalyst.

### DETAILED DESCRIPTION OF THE INVENTION

Specific examples of the compounds represented by the general formula (II) include 4-hydroxy-4′-iodobiphenyl, 4-hydroxy-4′-chlorobiphenyl, 4-hydroxy-4′-bromobiphenyl and derivatives thereof whose 4-hydroxyl group is converted to an acetoxy, benzoyloxy, methyl ether or ethyl ether group. Industrially preferred examples are 4-hydroxy-4′-bromobiphenyl and 4-acetoxy-4′-bromobiphenyl from the viewpoint of safety, hygiene, high yield and quality during preparing the same as well as purification treatments required in the method of the present invention.

As the inert organic polar solvents which may be used in the method of the present invention, there may be mentioned such primary alcohols as methanol, ethanol, propanol and butanol; such polyhydric alcohols as ethylene glycol, propylene glycol and glycerin; and lower alkyl ethers thereof and preferred are methanol, ethanol and ethylene glycol. The amount thereof is 1 to 10 times and preferably 1.5 to 3 times the weight of the compound of the general formula (II). In this respect, these inert organic polar solvent may be used in combination with water.

Examples of the hydroxides, carbonates or bicarbonates of alkali metals or alkaline earth metals include hydroxides, carbonates or bicarbonates of alkali metals or alkaline earth metals such as Na, K, Mg, Ca, Zn and Ba. Preferred are sodium hydroxide and potassium hydroxide. These inorganic substances are used in the form of fine powder or an aqueous solution. In general, it is preferred to use these as an aqueous solution having a concentration ranging from 1 to 50% by weight, in particular 5 to 20% by weight.

Examples of suitable palladium catalysts include those supported by a carrier such as active carbon, silica, alumina, silica-alumina, diatomaceous earth, zeolite, calcium carbonate, strontium carbonate and barium sulfate. The amount of palladium to be supported by such a carrier ranges from 0.1 to 20% by weight, preferably 0.5 to 10% by weight on the basis of the weight of the carrier. The amount of the catalyst used corresponds to the range of from 0.01 to 100 mmoles, preferably 1 to 50 mmoles per mole of the compound (II), expressed in amount of the metal atom. After the completion of the reaction, the catalyst may be separated and recovered to repeatedly use it in the reaction. If the activity thereof is lowered, the catalyst (together with the carrier) may be calcined, reduced and activated to regenerate the activity almost comparable to its initial level. Therefore, the manufacturing expenses are not so heavy if the loss of the catalyst during handling is reduced as low as possible.

The reaction temperature and time are properly selected according to the aforementioned reaction conditions, but the reaction temperature generally ranges from 50 to 200°C, preferably 80 to 150°C while the reaction time ranges from 1 to 12 hours, usually 2 to 6 hours.

The reaction pressure varies depending on the kinds of solvents used and the reaction temperature and the reaction can be performed at ordinary pressure or under pressure. The reaction atmosphere is not necessarily replaced with an inert gas or a reducing gas.

After the reaction, the reaction mixture is filtered to recover a mixture of the end product (salt) and the catalyst and an aprotic polar solvent such as N,N-dimethylformamide, N,N-dimethylacetamide or 1,3-dimethyl-2-imidazolidinone is added to the mixture to dissolve the end product and to thus recover and separate the catalyst from the end product. The polar solvent solution of the end product is precipitated by the addition of an acid such as diluted sulfuric acid or diluted hydrochloric acid and then the precipitates are filtered off. Alternatively, it is also possible to recover the end product by converting it (in the form of a salt) to its free state by the addition of an acid such as diluted sulfuric acid or diluted hydrochloric acid to the reaction mixture, adding an aprotic polar solvent thereto and heating to dissolve only the end product, then filtering off the catalyst and cooling the filtrate to crystallize the end product. The end product or the starting material (or the dehalogenated derivatives thereof) may further be recovered from the resultant mother liquor or the foregoing mother liquor resulting from the filtration of the precipitates formed by the addition of the acid.

The end product thus prepared is purified by, for instance, recrystallization from a proper solvent according to need.

In the general formula (II), if the substituent R represents an acyl group, it is sometimes observed that a deacylation reaction depending on the kinds of alkaline agents used and the reaction temperature selected. In particular, when R is an acetyl group, such a deacetylation reaction is simultaneously occurred and thus the compounds represented by the general formula (I) present in the reaction mixture are obtained in the form of dihydroxyl derivatives thereof (salts). Therefore, this simultaneous occurrence of the hydrolysis of the acyl group is rather advantageous on account of purposes and applications of the compounds represented by the general formula (I).

The method of the present invention will hereunder be explained in more detail with reference to the following working Examples. In the following Examples, the term "%" means "% by weight" unless otherwise specified.

### Example 1

There were charged, into a one liter-volume pressure reactor of SUS, 60.0 g(0.24 mole) of 4-hydroxy-4'-bromobiphenyl, 100 g of methanol, 300 g of 10% aqueous solution of sodium hydroxide and 13 g of a 5% palladium-carbon catalyst and the mixture was reacted at 120°C for 4 hours under stirring while applying a pressure ranging from 4.1 to 5.1 Pa x 10⁵ (4 to 5 atm./cm²). After cooling the reaction mixture to room temperature, the resultant solid content (a mixture of disodium salt of 4,4''' -dihydroxyquater-phenyl and the catalyst) was filtered off, followed by charging the solid content into a 1.5 liter-volume reaction flask, adding 1 liter of N,N-dimethylformamide, heating the solution with stirring and filtering it while it was still hot after one hour. The filtrate was cooled, followed by precipitating with the addition of a diluted sulfuric acid, filtering off the precipitates, washing with methanol and then drying the precipitates to obtain 24.5 g of 4,4'''-dihydroxyquater-phenyl as white crystalline powder (yield = 60.1%; melting point (M.P.) = 420°C; purity according to high performance liquid chromatography = 98.5%). This was recrystallized from N,N-dimethylformamide and the resultant purified product had M.P. of 440°C and purity of 99.9%.

8.4 g of a mixture of 4-hydroxybiphenyl and 4-hydroxy-4'-bromobiphenyl (the former = 66%; the latter = 34%) was recovered, from the mother liquor of the reaction mixture, by precipitation with an acid while 5.2 g of 4,4''' -dihydroxyquater-phenyl (purity = 98.4%) was recovered from the mother liquor resulting from the N,N-dimethylformamide solution, by precipitation with an acid. Total weight of the end product was 29.7 g and total yield thereof was 73.2%. The amount of the palladium-carbon catalyst recovered was 12.5 g (recovery = 96.2%; most of the loss thereof was due to handling).

### Example 2

The same procedures as in Example 1 were repeated, the 5% palladium-carbon catalyst recovered in Example 1 was repeatedly used while making up a deficiency with the fresh palladium-carbon catalyst and the reaction was repeated 5 times. The results thus obtained are summarized in Table 1 given below.

**Table 1**

| Repetition No. | Total Amount obtained (g) | Yield (%) | Purity (%) | Recovered^{*1)} starting material |
|---|---|---|---|---|
| 0^{*2)} | 29.7 | 73.2 | 98.5 to 98.4 | 8.4 |
| 1 | 30.0 | 74.0 | 99.7 to 98.0 | 8.4 |
| 2 | 29.5 | 72.7 | 99.1 to 98.2 | 8.3 |
| 3 | 28.3 | 69.8 | 97.4 to 99.2 | 8.9 |
| 4 | 28.0 | 69.0 | 99.0 to 98.9 | 9.0 |
| 5 | 27.5 | 67.8 | 99.6 to 98.5 | 9.3 |

| | | | | |
|---|---|---|---|---|
| *1) A mixture of 4-hydroxybiphenyl and 4-hydroxy-4′-bromobiphenyl. | | | | |
| *2) Example 1 | | | | |

### Example 3

The 5% palladium-carbon catalyst repeatedly recovered over five times in Example 2 was washed with water and dried at 70°C followed by raising the temperature to 300°C over 3 hours in a stream of nitrogen gas according to an ordinary manner, then substituting the nitrogen gas stream for a stream of hydrogen gas and maintaining the temperature at 360°C for 3 hours to regenerate the catalyst. As in Example 1, the reaction was carried out using 12.0 g of the resulting regenerated catalyst to which 1.0 g of the fresh catalyst was supplemented. Total amount of the product obtained was 30.8 g, the yield thereof was 75.9% and the purity was 99.1 to 99.8%. The total amount of the recovered 4-hydroxybiphenyl and 4-hydroxy-4′-bromobiphenyl was 8.3 g.

### Example 4

According in the same manner as in Example 1, 4,4‴-dihydroxyquater-phenyl was obtained utilizing 60.0 g (0.206 mole) of 4-acetoxy-4′-bromobiphenyl, 100 g of methanol, 300 g of 10% aqueous solution of sodium hydroxide and 13 g of 5% palladium-carbon catalyst. The total amount of the resultant product was 27.5 g, the yield thereof 79.0%, the purity 98.5 to 99.0% and M.P. 415 to 420°C.

### Example 5

There were charged, in a one liter-volume reactor, 60.0 g of 4-hydroxy-4'-bromobiphenyl, 150 g of ethylene glycol, 300 g of 10% aqueous solution of sodium hydroxide and 7.5 g of 5% palladium-carbon catalyst and the reaction was carried out at a reflux temperature (about 105°C) of the reaction mixture, for 5 hours with stirring (at ordinary pressure). The reaction mixture was cooled to room temperature, followed by adjusting pH to 3 by adding 25% diluted sulfuric acid and filtering off the resultant solids. To the solids there was added 1,000 ml of N,N-dimethylformamide, the solution was heated and was filtered at about 120°C. The filtrate was cooled to 15°C, the resultant crystalline deposits was filtered off, washed and dried to obtain 24.5 g of 4,4'''-dihydroxyquater-phenyl as white crystalline powder. The purity thereof was 99.1% and M.P. was 420°C. There was recovered 4.8 g of 4,4'''-dihydroxyquater-phenyl from the mother liquor resulting from the filtration of the precipitates formed by the addition of the acid. The purity thereof was 99.0% and M.P. was 420°C. The total amount of the end product was 29.3 g and the overall yield was 72.1%.

### Example 6

The reaction was performed under pressure according to the same procedures as in Example 1 except that 63.0 g (0.24 mole) of 4-methoxy-4'-bromobiphenyl were used instead of 60.0 g of 4-hydroxy-4'-bromobiphenyl, the resultant reaction mixture was cooled to 15°C and was filtered. To the solid filtered off there was added one liter of N,N′-dimethylformamide, followed by heating the solution, filtering the solution while it was still hot to remove the catalyst, cooling the filtrate to 15°C, filtering it and drying to obtain 24.5 g of 4,4‴-dimethoxyquater-phenyl as a white crystalline substance. The yield thereof was 55.8%; M.P. was 336°C and the purity thereof was 98.5%.

### Example 7

To the same apparatus as used in Example 1 there were charged 60.0 g of 4-acetoxy-4′-bromobiphenyl instead of 4-hydroxy-4′-bromobiphenyl, 150 g of ethylene glycol, 300 g of 10% aqueous solution of sodium hydroxide and 12.4 g of 5% palladium-carbon catalyst and they were reacted at the reflux temperature (about 105°C) of the mixture, at ordinary pressure for 5 hours with stirring. After the reaction, the same procedures as in Example 1 were repeated to obtain 20.8 g of 4,4‴-dihydroxyquater-phenyl as white crystalline powder. Yield 50.9%; purity 99.7% and M.P. 420°C. In addition, there was recovered 4.8 g of 4,4‴-dihydroxyquater-phenyl (purity 98.8%) from the mother liquor resulting from the filtration of the precipitates formed by the addition of the acid. The total amount of the end product recovered was 25.6 g and the overall yield was 62.6%.

## Claims

1. A method for preparing 4,4‴-dihydroxyquater-phenyl or derivatives thereof represented by the following general formula (I): 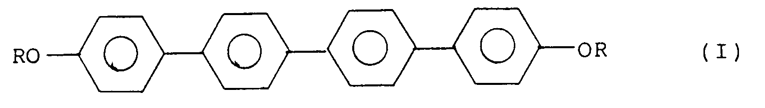 wherein R represents a hydrogen atom or an alkyl having 1 to 4 carbon atoms or an acyl group which comprises dehalogenating and dimerizing a 4-hydroxy-4′-halogenobiphenyl or derivatives thereof represented by the following general formula (II): 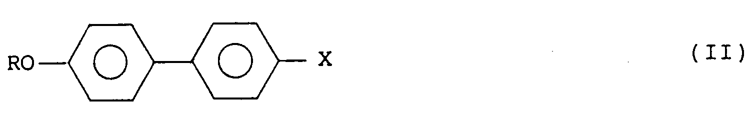 wherein R is the same as that defined above and X represents a halogen atom in an inert organic polar solvent, in the presence of at least one member selected from the group consisting of hydroxides, carbonates or bicarbonates of alkali metals or alkaline earth metals, in the absence of carbon monoxide and utilizing palladium as a catalyst.

2. A method as set forth in claim 1 wherein the dehalogenation and dimerization were performed in the presence of water in combination with the inert organic polar solvent.

3. A method as set forth in claim 1 wherein the dehalogenation and dimerization were performed at a temperature ranging from 50 to 200°C.

4. A method as set forth in claim 1 wherein the palladium catalyst is a palladium catalyst supported by a carrier and it is used in an amount of 0.01 to 100 mmole per mole of the compound (II) used, expressed in amount of the metal atom.

## Patentansprüche

1. Verfahren zur Herstellung von 4,4'''-Dihydroxyquaterphenyl oder Derivaten davon, die durch die folgende allgemeine Formel (I) dargestellt werden: 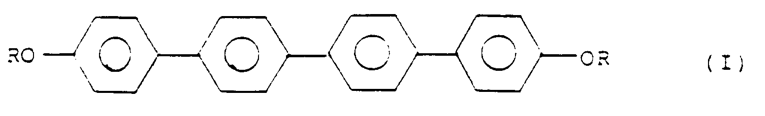 in der R ein Wasserstoffatom oder eine Alkylgruppe mit 1 bis 4 Kohlenstoffatomen oder eine Acylgruppe darstellt, welches die Dehalogenierung und Dimerisierung von 4-Hydroxy-4-halogenbiphenyl oder dessen Derivaten, die durch die folgende allgemeine Formel (II) dargestellt werden: 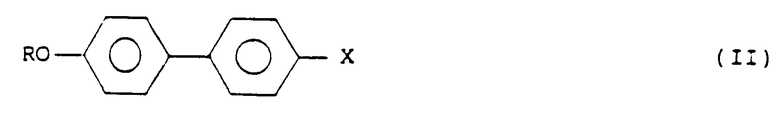 in der R wie vorstehend definiert ist und X ein Halogenatom darstellt, in einem inerten, organischen, polaren Lösungsmittel in Anwesenheit wenigstens einer Verbindung, ausgewählt aus der Gruppe bestehend aus Hydroxiden, Carbonaten oder Bicarbonaten von Alkali- oder Erdalkalimetallen, in Abwesenheit von Kohlenmonoxid und unter Verwendung von Palladium als Katalysator umfaßt.

2. Verfahren nach Anspruch 1, bei dem die Dehalogenierung und Dimerisierung in Anwesenheit von Wasser in Kombination mit dem inerten, organischen, polaren Lösungsmittel durchgeführt werden.

3. Verfahren nach Anspruch 1, bei dem die Dehalogenierung und Dimerisierung bei einer Temperatur von 50 bis 200°C durchgeführt werden.

4. Verfahren nach Anspruch 1, bei dem der Palladiumkatalysator ein auf ein Trägermaterial aufgetragener Palladiumkatalysator ist und in einer Menge von 0,01 bis 100 mMol pro Mol Verbindung (II), ausgedrückt als Menge des Metallatoms, verwendet wird.

## Revendications

1. Procédé de préparation de 4,4'''-dihydroxyquaterphényle ou de dérivés de celui-ci représentés par la formule générale (I) suivante : 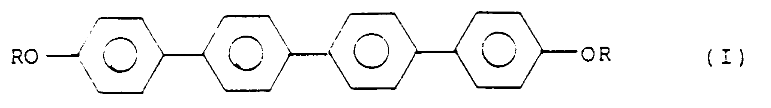 dans laquelle R représente un atome d'hydrogène ou un groupement alkyle en C₁-C₄ ou un groupement acyle, qui comprend la déshalogénation et la dimérisation d'un 4-hydroxy-4'-halogénobiphényle ou de dérivés de celui-ci représentés par la formule générale (II) suivante : 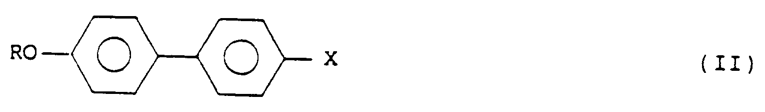 dans laquelle R est tel que défini ci-dessus et X représente un atome d'halogène ; dans un solvant organique polaire, en présence d'au moins un des éléments du groupe constitué des suivants : hydroxydes, carbonates ou bicarbonates de métaux alcalins ou de métaux alcalino-terreux, en absence de monoxyde de carbone et en utilisant du palladium comme catalyseur.

2. Procédé selon la revendication 1, dans lequel la déshalogénation et la dimérisation ont été effectuées en présence d'eau en combinaison avec le solvant polaire organique inerte.

3. Procédé selon la revendication 1, dans lequel la déshalogénation et la dimérisation ont été effectuées à une température comprise entre 50 et 200°C.

4. Procédé selon la revendication 1, dans lequel le catalyseur au palladium est un catalyseur au palladium supporté par un support et est utilisé à raison de 0,01 à 100 mmol/mole du composé (II) utilisé, exprimé en quantité d'atomes de métal.
